Europäisches Patentamt

**European Patent Office**

**Office européen des brevets**

(11) Publication number: **0 092 208**
A2

(12) # EUROPEAN PATENT APPLICATION

(21) Application number: **83103694.2**

(22) Date of filing: **16.04.83**

(51) Int. Cl.³: **B 01 D 53/04,** C 07 C 29/76, C 07 C 31/08

(30) Priority: **19.04.82 US 369836**

(43) Date of publication of application: **26.10.83** Bulletin 83/43

(84) Designated Contracting States: **AT BE CH DE FR GB IT LI LU NL SE**

(71) Applicant: **UNION CARBIDE CORPORATION, Old Ridgebury Road, Danbury Connecticut 06817 (US)**

(72) Inventor: **Frost, Albert Carl, 73 New York Avenue, Congers, N.Y. 10920 (US)**

(74) Representative: **Eggert, Hans-Gunther, Dr., Räderscheidtstrasse 1, D-5000 Köln 41 (DE)**

(54) Improved gas separation process.

(57) The separation of gas mixtures in commercial sized adsorption systems is carried out with a carefully limited difference in loading of the adsorbed component on the bed between the start and the end of the desorption step. Under such conditions, each succeeding adsorption step will be carried out so that an adsorption/heating front is caused to advance sufficiently ahead of a slower moving cooling front that thus serves to cool only the feed end of the bed to the inlet gas mixture temperature. The heat of adsorption is thereby retained in the bed between these two fronts at a temperature tolerable to the gas mixture and to the adsorption bed and is available for the following desorption step.

EP 0 092 208 A2

## IMPROVED GAS SEPARATION PROCESS

### Background of the Invention

Field of the Invention - The invention relates to the separation of gas mixtures. More particularly, it relates to the retention and use of the heat of adsorption generated during the selective adsorption of a component of a gas mixture.

Description of the Prior Art - In the separation of normally liquid mixtures, distillation is commonly employed when the components of the mixtures have appreciably different boiling points and the components are not capable of forming an azeotrope. If the components are capable of forming an azeotrope or if their relative volatility is close to one, however, the desired separation may require so many distillation trays and/or such a high reflux ratio as to render the distillation unsuitable or very expensive in terms of energy consumption and capital costs. In the drying of 190+ proof ethanol, azeotropic distillation with a third component, such as benzene, cyclohexane, or normal pentane is commonly employed to remove water from the ethanol. The extraction process likewise involves high capital costs and the expenditure of large amounts of energy. As a result, other techniques are desired as alternatives to the use of such distillation or extraction operations.

One such technique involves the passing of the liquid mixture through a molecular sieve bed capable of selectively adsorbing one component from the mixture. The adsorbed component, as well as the residual liquid retained in the macropores and in the interstitial spaces between the adsorbent particles, are subsequently removed from the bed by means of heat. For this purpose, a furnace and a recycle compressor must be provided so that hot purge gas can be passed to the bed to desorb and remove the adsorbed component from the bed before additional quantities of the mixture are passed to the bed in a continuous, cyclic operation. The need for such heat and recycle compressor capability is a considerable element of expense, however, detracting from the commercial desirability of this approach.

It has also been proposed to employ a vapor or gas feed approach in which a normally liquid mixture is passed in the vaporized state to an adsorption bed for the selective adsorption of one or more of the components thereof. In such an approach, no heat is required for desorption, and the requirements for a recycle compressor can be reduced or even eliminated depending upon the particular adsorption-desorption technique employed. The problems associated with the achieving of high purity products from azeotropic mixtures, as in the drying of alcohols, is discussed in the Skarstrom et al patent, U.S. 3,122,486. This patent relates particularly to the combination of a distillation column with a heatless fractionator, the invention described therein being an adaptation of the process and apparatus of the Skarstrom patent, U.S. 2,944,627. In the technique of the latter patent, one adsorption bed is counter-currently depressurized and a portion of the product effluent from another bed is commonly employed as a purge gas to facilitate removal of the adsorbed

component from the depressurized bed. Skarstrom teaches the conservation of heat evolved during the adsorption cycle by the rapid cycling of the adsorption and desorption-purge steps so as to reduce the flow of heat through the bed and through the walls of the adsorption vessel into the surrounding atmosphere. In this manner, heat produced during the adsorption step can be substantially utilized during the desorption-purge step as discussed in Column 6, Line 7 - Column 7, Line 16. Skarstrom indicates that the particular cycle times depend on a variety of circumstantial factors, such as the adsorbent employed, the height of the bed, the gas mixture to be separated and the like.

The loss of heat through the walls of the adsorption vessel, contributing to the need for the rapid cycling technique proposed by Skarstrom, is a significant factor in operations carried out in the scale employed by Skarstrom. Thus, Skarstrom refers specifically to adsorption beds 2.875 inches in diameter. At such a size, heat loss through the walls of the vessel can contribute to an appreciable loss of heat generated during the adsorption step. In commercial size adsorption beds having a diameter of at least about one foot and generally considerably larger, on the other hand, heat loss through the walls of the adsorption vessel is not a significant factor as it would be in the smaller sized vessels employed by Skarstrom. The heat loss through the vessel walls, as will be appreciated by those skilled in the art, is inversely proportional to the square of the diameter. Thus, large, commercial sized adsorption vessels are not

12808

subjected to the same problem of heat loss through the vessel walls as is encountered in the practice of the Skarstrom technique in the relatively small sized adsorbers disclosed in the Skarstrom patent.

It is nevertheless desirable, in commercial size adsorption-desorption gas separation operations, to utilize the heat generated during each adsorption step in a manner reducing the overall cost of the process. It has been found, however, that the retention of the heat of adsorption within a commercial size adsorption bed is dependent upon factors not totally consistent with the conventional operation of adsorption systems to achieve optimum product purity. It is generally considered desirable to attain high adsorbate loadings of the desired components during the cyclic operations by using relatively high adsorption pressures and/or low desorption pressures. It is entirely possible to operate in such an apparently desirable manner, but by doing this, the heat of adsorption is usually effectively removed from the bed with the product effluent. It is desired in the art, however, to develop techniques to enable the heat of adsorption to be retained in the bed and utilized in the adsorption-desorption processing sequence. The drying of alcohols is an example of the industrial processing applications in which such techniques could be employed to appreciable overall advantage.

It is an object of the invention, therefore, to provide an improved process for the selective adsorption of a component of a gas mixture in a commercial sized adsorption bed.

It is another object of the invention to

12808

provide a selective adsorption process adapted for the retention of the heat of adsorption within the adsorption bed.

With these and other objects in mind, the invention is hereinafter described in detail, the novel features thereof being particularly pointed out in the appended claims.

## Summary of the Invention

By limiting the amount of adsorbed component removed from a commercial size adsorption bed during each desorption step, the heat generated during the next succeeding adsorption step can be effectively retained within the bed at a temperature tolerable to said gas mixture and to the adsorbent material. Desorption can be achieved, within the desirable limits, by means of purge gas or by a pressure swing adsorption process. In alcohol-water drying operations, the desorbate can advantageously be recycled to the distillation column from which a concentrated alcohol-water feed stream is passed to said adsorption bed system.

## Brief Description of the Invention

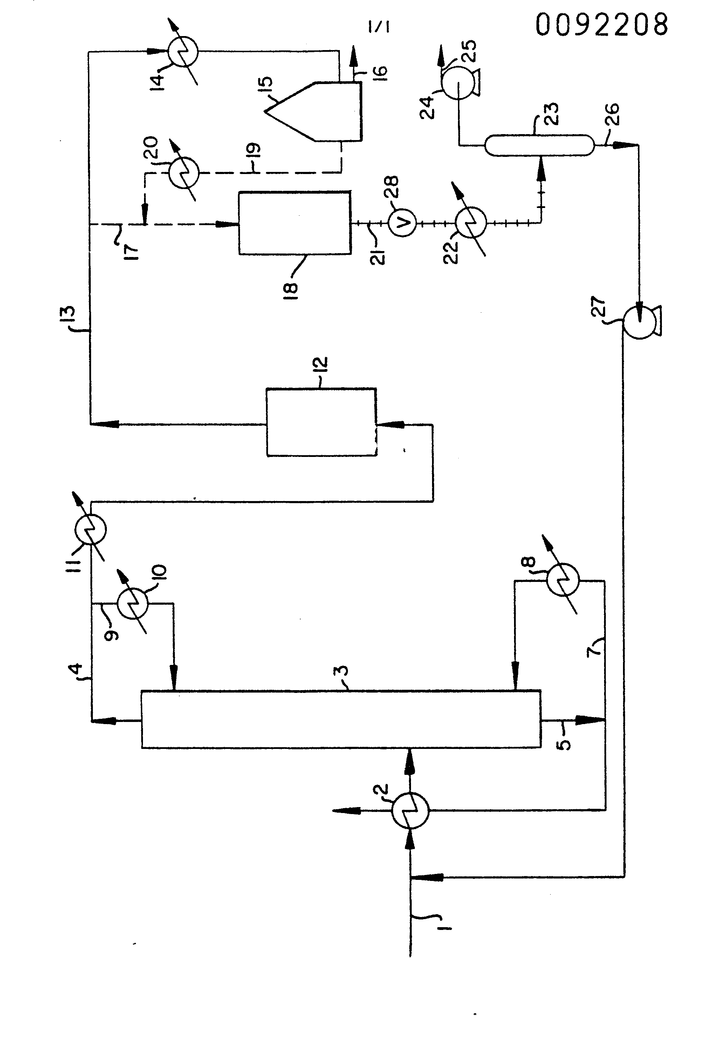

The invention is further described with reference to a single figure drawing illustrating the process flow diagram of a preferred embodiment of the invention for achieving the drying of ethanol.

## Detailed Description of the Invention

The objects of the invention are accomplished by carefully limiting the amount of desorbate removed from an adsorption bed during each desorption step of a cyclic adsorption-desorption process for the separation of a gas mixture. In

12808

0092208

this manner, the next succeeding adsorption step will be carried out so that an adsorption/heat front will be caused to advance sufficiently ahead of a slower moving, lagging cooling front that will thus serve to cool only the feed end of the bed to the inlet gas mixture temperature. The heat of adsorption will then be retained in the bed between the two fronts at a temperature tolerable to the gas mixture and to the adsorbent material. The retention of said heat is highly desirable as it then becomes available for use in supplying the heat for the following desorption step. In this manner, the invention contributes to the overall technical-economic feasibility of achieving desired gas separations by means of cyclic adsorption-desorption processes.

In cyclic adsorption processes for the separation of gas mixtures in commercial size adsorption beds, there will be, as indicated above, no significant loss of the heat of adsorption by passage through the walls of the adsorption vessel within typical cyclic operation times. A heat front will form in the bed, however, and travel towards the end of the bed during each adsorption step.

In high cyclic loading operations previously considered desirable, the heat front will advance to the product end of the bed and beyond, i.e. with the product effluent, ahead of a slower moving adsorption/cooling front that moves from the feed end to the product end of the bed as each adsorption step is carried out in the bed. Thus, the heat of adsorption is not retained in the bed and is not available for the subsequent desorption step in such instances. In the favorable circumstances ensured by the practice of the invention, on the other hand, the heat front is caused to advance with the adsorption front towards, but not beyond the end of the bed, and ahead of a relatively slower moving, lagging cooling front. More particularly, the adsorption/heat front is caused to advance sufficiently ahead of said relatively slower moving, lagging cooling front that thus serves to cool only the feed end of the bed to the inlet gas mixture temperature. The heat of adsorption will thereby be retained in the bed between the adsorption/heat front and the lagging or trailing cooling front at a temperature tolerable to said gas mixture and to said adsorbent material.

The commercial sized adsorption systems to which the invention is directed and limited have bed diameters of at least about one foot and contain an adsorbent capable of selectively adsorbing one component of a gas mixture from another. The diameter of such beds is generally on the order of from one to about twelve feet, with diameters of from about two to about eight feet being commonly employed. Such adsorption beds will be used for the separation of a gas mixture containing a relatively more polar gas component present in admixture with a relatively less polar gas component. It is within the scope of the invention to have the adsorption beds adapted for the adsorption either of the more polar gas component or of the less polar gas component. Those skilled in the art will appreciate that adsorbent materials are available for selectively adsorbing one or the other of said components from a gas mixture.

In the practice of the invention, the gas

12808

mixture to be separated is introduced to the feed end of the commercial sized adsorption bed, and non-adsorbed component is withdrawn from the product end of the bed. The introduction of the gas mixture to the bed is terminated while a relatively fast moving adsorption/heating front formed upon the adsorption of the adsorbed component remains in the bed. The adsorbed component is thereafter desorbed from the bed and countercurrently discharged from the feed end of the bed. Contrary to the general desire to desorb the bed as fully as possible in preparation for carrying out the next succeeding adsorption step with additional quantities of the feed gas mixture, the process of the invention is carried out so that the difference in loading of the adsorbed component in the bed between the start and the end of said countercurrent discharge step is maintained sufficiently low so that, during the next succeeding adsorption step, an adsorption/heating front will be caused to advance ahead of a relatively slower moving, lagging cooling front. By carefully limiting the difference in loading on the bed between the end of the adsorption step and the end of the desorption step, the difference in the rate of advance of the more rapidly advancing adsorption/heat front and the slower moving cooling front, which thus serves to cool the feed end of the bed to the inlet gas mixture temperature, will be such that the heat of adsorption will be advantageously stored in the bed, between the two fronts, at a desired temperature that the gas mixture being fed to the bed and the adsorbent material in the bed can reasonably tolerate. It should be understood that the difference in loading

12808

as herein described refers to the equilibrium section of the bed only, and not to the entire bed.

It should be noted that the permissible difference in loading of the adsorbed component on the bed, which is maintained low in accordance with the practice of the invention, will vary depending on the overall processing conditions that pertain to any given application. Thus, (1) the gas mixture to be separated, (2) the adsorbent material employed in the bed, (3) and the degree of feed stream separation desired are collectively the primary factors that define that extent of the difference in loading that should be used for that application. The specific loading difference limits pertaining to the practical ethanol drying application referred to above are disclosed below, together with a description of the basic relationships used in this application, or for any other application, to determine the permissible loading difference limitations necessary to achieve the benefits of the invention.

In the ethanol drying application, a gas mixture comprising about 7.5 weight % water is commonly available as a feed stream to the adsorption-desorption system herein described. In accordance with the practice of the invention, the difference in loading of water on the bed between the end of the adsorption step and the end of the countercurrent discharge step should be within the range of from about 0.1 to about 3.4 lb. of adsorbed water per 100 lbs. of dry adsorbent for practical operating purposes. With such a limited difference in water loading, the heat of adsorption can conveniently be retained in the bed between the heating and cooling fronts using essentially undiluted adsorbent having a typical heat capacity of generally on the order of about 0.25 BTU/lb. of adsorbent -°F in the drying of said about 92.5 wt. % ethanol/7.5 wt % water feed stream to a product having less than about 1% water by weight. In preferred embodiments of this application of the invention, said loading difference is maintained within the range of from about 0.5 to about 2.5 lb of adsorbed water per 100 lb. of dry adsorbent so as to achieve a reasonable loading of adsorbate on the bed while, at the same time, ensuring moderation in the exothermic temperature rise between the leading adsorption/heating front and the lagging cooling front in the bed during each succeeding adsorption step. It will be understood that at very low differences in loading, the adsorptive capacity of the bed will not be utilized to its full potential. As a result, uneconomically large adsorption beds would be required. If, on the other hand, larger loading differences approaching the upper limit of the permissible range were employed, the exothermic heat of adsorption would result in a large temperature rise between the leading adsorption/heating front and the lagging cooling front in the bed that may result in a shortening of the effective life of the adsorbent and/or the initiation of feedstock degradation reactions. This will be appreciated from a further consideration of the factors affecting the positioning of the heating and cooling fronts during the adsorption operation.

The relative positions of the heating and cooling fronts described above can be approximated by the following ratio based upon mass and heat flow considerations pertaining during the course of the adsorption step, namely $(Cpg)(\Delta W)/(Cps)(\Delta Y)$, wherein Cpg represents the heat capacity of the unadsorbed component of the gas mixture expressed in terms of BTU's per lb. of the unadsorbed component(s)-degree Fahrenheit; Cps is the heat capacity of the adsorbent bed solids in terms of BTU's per lb. of solids-degree Fahrenheit; $\Delta W$ is the difference in loading of adsorbate in the bed between the start and the end of the desorption step in terms of lb. of adsorbate per 100 lb. of dry adsorbent; and $\Delta Y$ is the difference between the amount of adsorbable component in the feed and in the product effluent, in terms of lbs. of said adsorbable component per lb. of the non-adsorbed product gas effluent. It should be noted that although the $\Delta W$ will be kept low in accordance with the teachings herein, the residual loading at the end of the desorption step may, nevertheless, be relatively high.

It has been determined that when the indicated ratio is in excess of one, the heat front formed in the bed during the adsorption step will travel rapidly through and out of the bed in advance of a slower moving, trailing adsorption/cooling front. In this case, the heat of adsorption will not be retained in the bed and will not be available for use during the desorption step. By maintaining the ratio at less than one, however, the heat of adsorption can be retained within the bed between a faster moving adsorption/heating front and a relatively slower moving, lagging cooling front. As the ratio reaches one from either direction, the retained heat of adsorption will be found to cause a nearly unlimited temperature rise to occur as the retained heat will be confined within an extremely limited portion of the bed as the system crosses over from one sequence of advancing fronts to the other. In the drying of the 92.5 wt. % ethanol-7.5 wt. % water mixture referred to above wherein said Cpg is 0.49 BTU's per lb. of the unadsorbed component -°F, Cps is 0.25 BTU's per lb. of adsorbent solids -°F; and $\Delta$Y is 0.07 lb. of adsorbable water per lb. of non-adsorbable ethanol and water, the difference in loading, or $\Delta$W, is then found to be about 3.5 lb. of adsorbed water per 100 lb. of dry adsorbent when the ratio is one. In order to avoid extremely high bed temperatures, the permissible difference in loading in this case is less than said 3.5 and is within the operable ranges referred to above. At such limited differences in loading, the ratio will be less than one so that the heat of adsorption will be retained in the bed between the two advancing fronts and will be at a temperature tolerable to the gas mixture being treated and to the adsorbent material itself. It should be noted that the $\Delta$Y of 0.07 lb. of adsorbable water per lb of non-adsorbable ethanol and water is based upon a typical circumstance in which the ethanol product effluent from the pressure swing adsorption system contains approximately 1 wt. % water. If all of the water were removed from the feed stream to the system, however, $\Delta$Y would be 0.081. In this case, $\Delta$W would by 4.1 lbs. of adsorbed water per 100 lb. of adsorbent material when the indicated ratio is one. Under such circumstances, the permissable loading difference would extend from about 0.1 up to about 4.0 lbs. per 100 lb. of adsorbent material to achieve the indicated benefits of the invention.

12808

From the description above, those skilled in the art will appreciate that various factors will influence the permissible limits of the difference in adsorbate loading in particular applications of the invention. In the ethanol drying application, for example, an increase in the heat capacity of the adsorbent bed particles or an increase in the $\Delta Y$ component will result in an allowable increase in the permissable adsorbate loading on the bed, i.e. $\Delta W$, to maintain the relative rates of the more rapidly moving adsorption/heating front and the trailing cooling front. Such an increase in the heat capacity of the bed can be achieved, for example, by diluting the adsorbent particles with higher heat capacity steel balls. An increase in $\Delta Y$ might result from the passing of a less concentrated feed gas mixture to the adsorption-desorption system. An ethanol-water feed stream having more than about 7.5% by weight water might be passed to the system, for example, with the same final product purity to be maintained in the system. It will also be appreciated that the difference in loading of the adsorbed component on the bed at the start and at the end of the desorption step can likewise be determined for similarly conserving the heat of adsorption within the bed in any other gas separation application of the invention. While the specific heat capacities and the $\Delta Y$ may change from one application to another, the difference in loading will be limited, in each instance, so that the above-indicated ratio is maintained reasonably under a value of one so that the stated benefits of the invention can be achieved.

Upon determination of a desirable $\Delta W$ for a

given application by the heat front and temperature rise considerations discussed above, the operative conditions used to obtain this result can readily be determined by those skilled in the art from the adsorption isotherms pertaining to a particular adsorbate/adsorption system. Such isotherms include the isotherm for the feed temperature and for the maximum bed temperature, for the partial pressures corresponding to the two bed loadings chosen to define said $\Delta$W, i.e. the adsorbate partial pressure for the feed loading and the adsorbate partial pressure for the residual loading. For the ethanol/ water application referred to above using the pressure swing adsorption approach hereinafter described in further detail, for example, the 325°F isotherm for a zeolite 3A/water will define the residual loading on the bed material for a desirable 1 psia lowest desorption pressure. Examination of the 435°F isotherm at a loading greater than the residual loading by the chosen $\Delta$W, for example by 1 lb. of water per 100 lbs. of said zeolite 3A, indicates a partial pressure that, when divided by the mole fraction of water in the feed, suggests a desired adsorption pressure of 50 psia. The operating conditions for achieving the desired limitation in the difference in bed loading, between the start and the end of the countercurrent discharge step, can thus be readily determined in a similar manner for any given gas separation application of the invention.

In the practice of the invention, any suitable adsorbent material capable of selectively adsorbing one or more components of a gas mixture may be employed. One commonly used adsorbent is the well known zeolite 3A, which is the potassium-exchanged form of zeolite A that has micro-pore openings of about 3 Angstroms in diameter. The sodium-exchanged form, i.e. zeolite 4A or the calcium-exchanged form, i.e. zeolite 5A, can also be employed. The type 3A zeolite structure, it should be noted, has a relatively small pore openings and a very large capacity for the adsorption of water. As ethanol and other organic compounds are not able to effectively enter these small pore openings, such materials can only be adsorbed on the very limited outer surface area of the zeolite, constituting as little as about 1% of the total available surface area of the adsorbent. Selectivity is thus achieved in such instances by molecular size differences. It should be noted that, in the event that water were admixed with an even more polar component than said water, but one that could not effectively enter the 3A zeolite structure, then the zeolite would still selectively adsorb the water even though it would be the less polar component.

Other suitable, commercially available adsorbents include the sodium- and calcium-exchanged forms of zeolite X and of the more thermally stable zeolite Y, and the mordenite, erionite, clinoptilolite and chabazite ores as well as new alumino-phosphate zeolites, such as the ALPO-20 zeolite. While the zeolite 3A achieves selective adsorption principally on the basis of molecular size differences, it will be appreciated that the various other adsorbents referred to herein, and others known in the art, achieve the desired selectivity principally by a difference in polarity, said adsorbents being adapted to adsorb

12808

preferentially either the more polar or the less polar component or components of a gas mixture that can penetrate into the inner structure of the adsorbent. Although zeolite 3A may not possess very long term stability under the continuous, high temperature high-water vapor conditions encountered, it is nevertheless a generally desirable adsorbent for use in the ethanol drying application. Other suitable adsorbents that may tend to have greater long term stability nevertheless generally have somewhat lower selectivity. In any event, if the selectively adsorbed water, as recovered, has a significant amount of ethanol associated therewith, it is convenient to recycle the recovered water-ethanol stream to an ethanol-water distillation column as is described below with respect to preferred embodiments of the invention.

As noted above, the invention can be employed for the separation of the more polar or the less polar component of a gas mixture. It will be understood that the term "component" in this regard is used to denote either a single component or two or more components that may be advantageously separated from another component or components of a gas mixture. While the invention may be used for the separation of any such gas mixture for which a suitable adsorbent is available, the invention is particularly suited for the treatment of gas mixtures capable of forming an azeotrope or mixtures containing components having a relative volatility close to one, for the reasons referred to above. The invention has particular application in the separation of water as the more polar component of a gas mixture containing one or more organic compounds as the less polar component. Practical gas mixtures of this type will commonly contain at least about 2.5 weight % water. The organic compounds in such instances will typically comprise primary alcohols containing from 2 to 5 carbon atoms, with the above-mentioned ethanol and water admixture being a particularly practical application for the practice of the invention. While such admixtures will generally comprise from about 2.5 to about 30% by weight water, with the adsorption bed typically adapted for the selective adsorption of water, a feed gas stream containing about 92.5 wt. % ethanol and about 7.5 wt. % water is the most usual feed stream generally available for ethanol drying in commercial operations. The adsorption-desorption sequence is carried out, in the practice of the invention, so that the difference in loading of water on the bed, between the end of the adsorption step and the end of the countercurrent desorption step, is within the limits set forth above or under such modified limits as may be acceptable or required in keeping the heat of adsorption retained in the bed between an advancing, faster moving adsorption/heating front and a trailing cooling front.

In the practice of the invention, the controlled difference in loading of the adsorbed component on the bed between the end of the adsorption step and the end of the desorption step may be carried out by passing a non-sorbable purge gas, e.g. nitrogen, helium, hydrogen, carbon dioxide, methane or the non-adsorbed component, to the product end of the bed at a temperature and pressure essentially the same as that of the feed

12808

end of the bed. The purge gas and the desorbed component are discharged from the feed end of the bed. The cost of the purge compressor and the cost of circulating the purge gas, including its compression, its cooling to condense the non-adsorbed component therefrom, and its reheating to the desired purge temperature, represent, however, considerable elements of expense. Accordingly, it is generally desirable to develop an alternate approach to enhance the technical and economic feasibility of employing the subject adsorption-desorption process in commercial size units in accordance with the practice of the invention.

It has been found that the invention can preferably be carried out by the use of a pressure swing adsorption technique that can be adapted for the convenient and effective control of the bed loading difference during the desorption step. Thus, the feed gas mixture is introduced to the feed end of an adsorption bed at a higher adsorption pressure, and the desorption and countercurrent discharge of the desorbed component from the feed end of the bed are accomplished by countercurrently depressurizing the bed so as to release the adsorbed component from the adsorbent and to discharge said released component from the feed end of the bed. The bed is thereby depressurized to a lower final desorption pressure. The final desorption pressure, in accordance with the practice of the invention, is maintained sufficiently high so as to assure achieving the desired limitation on the difference in loading of the adsorbed component on the bed between the end of the adsorption step and the end

12808

of the desorption step.  In this manner, the heat of adsorption is retained in the bed at a temperature tolerable to the adsorbent material and to the components of the feed gas mixture.  The pressure swing adsorption embodiment will generally include, prior to said countercurrent depressurization, cocurrently depressurizing the bed to an intermediate pressure so as to release void space gas comprising non-adsorbed component from the discharge end of the bed.  This step will be advantageously carried out so as to advance the leading adsorption/heating front toward, but not past, the discharge end of the bed.  Furthermore, after the succeeding countercurrent depressurization has been completed, the bed can then be repressurized with non-adsorbent product effluent.  Further description of the pressure swing adsorption technique will be appreciated from a consideration of the embodiment of the invention illustrated in the accompanying drawing.

In the drawing, a system for carrying out the pressure swing adsorption process of the invention, as integrated with a prefractionator, is shown with respect to the drying of ethanol available in a dilute ethanol-water feed stream. Such a feed stream enters the system through line 1 and is heated in heat exchanger 2 prior to passage to an ethanol-water distillation column, or prefractionator, 3 from which a concentrated ethanol-water vapor stream is removed through line 4.  Water removed from the bottom of column 3 through line 5 is passed in line 6 through heat exchanger 2 and to discharge from the system.  A portion of said water in line 5, however, is diverted through line 7 to steam reboiler 8 from which it is passed back as a vapor to the lower portion of column 3 as in conventional fractionating practice. Similarly, a portion of the overhead vapor in line 4 is passed in line 9 through condenser 10 from which it is refluxed back to the upper portion of column 3.

The remaining overhead vapor in line 4 is passed to super-heater 11 and therefrom into adsorption bed 12. Effluent vapor from bed 12 is withdrawn through line 13 containing condenser 14 for condensing product effluent, e.g. dried ethanol, that is collected in surge tank 15 from which dried ethanol product liquid is removed through line 16. A portion of the vaporized product effluent from adsorption bed 12 can be diverted from line 13 for passage through line 17 for use in the repressurization of second adsorption bed 18 in the advantageous two-bed pressure swing adsorption system illustrated in the drawing. Alternatively, a portion of the product effluent stored in surge tank 15 can be withdrawn through line 19 and passed through vaporizer 20 for use in the repressurization of bed 18.

Adsorption bed 18 is illustrated for convenience as adapted for countercurrent desorption and countercurrent repressurization. It will be appreciated, however, that the overall system will include means so that both bed 13 and bed 18 can be passed through the adsorption step at higher pressure with product effluent withdrawal, cocurrent depressurization with additional product effluent recovery, countercurrent desorption and repressurization as the overall continuous processing sequence is carried out on a cyclic basis in each bed. Typically, one bed will be on its adsorption step while the other bed is undergoing the remaining steps of the processing sequence. As shown, the desorbed component is countercurrently discharged from the feed end of bed 18 through line 21 that contains condenser 22 adapted to condense the released desorbate material by external heat exchange using water or other suitable cooling media. The condensed desorbate leaves said condenser 22 and passes further in said line 21 to separator 23 from which non-condensible gases present in the desorbate are separated from said condensed desorbate. A small vacuum pump 24 is provided to draw such non-condensible gases from the separator through line 25 for discharge from the system. The condensed desorbate in separator 23 can readily be recycled to column 3 if it contains significant amounts of ethanol, thus providing a highly desirable integration of the overall process. Thus, the condensed desorbate can be withdrawn from separator 23 through line 26 and pumped by means of pump 27 for recycle back to column 3 together with additional quantities of the dilute ethanol-water feed stream to the system. It should be noted in the drawing that the flow stream line 4 entering vessel 12, and line 13 leaving said vessel, are solid lines representing the adsorption and cocurrent depressurization steps as carried out in said vessel 12 at particular sequential times in the overall process. The hatched line 21 leaving vessel 18 denotes the countercurrent desorption step carried out in said vessel 18 during a portion of the adsorption step in vessel 12. Similarly, the dotted lines 17 and 19 denote the repressurization of vessel 18 during the final portion of the adsorption step in said vessel 12.

In carrying out the process of the invention in the illustrated system, the dilute ethanol-water feed stream will typically contain about 10% ethanol and 90% water by weight, with prefractionator distillation column 3 commonly concentrating the feed stream to on the order of about 92.5 wt. % ethanol and about 7.5 wt. % water by weight. This concentrated stream is the feed gas mixture to the pressure swing adsorption system shown in the drawing. In embodiments in which zeolite 3A or other suitable adsorbent having a heat capacity of generally about 0.25 BTU/lb. of adsorbent -°F is employed, the countercurrent desorption step will be carried out so that the difference in loading of water on the bed between the end of the adsorption step and the end of the countercurrent release and discharge step is from about 0.1 to about 3.4, preferably about 0.5 to about 2.5 lb. of water per 100 lb. of dry adsorbent so as to achieve a desirable balance of reasonable loading of the bed and to ensure moderation of the exothermic temperature rise during each succeeding adsorption step, wherein the heat of adsorption is maintained within the bed.

The feed gas mixture is conveniently passed to the adsorption system for adsorption at the prefractionator pressure of from about 35 to about 100 psia and at a superheated temperture of from about 300°F up to about 375°F. Each bed is desirably cocurrently depressurized to an intermediate pressure level within the range of from about 10 to about 50 psia before countercurrent depressurization to a final lower desorption pressure of from about 0.1 to 10 psia. In preferred embodiments of this application of the invention, the adsorption pressure is about 35-50 psia, said intermediate pressure is about 10-20 psia and said final lower desorption pressure is about 1-3 psia.

The final lower desorption pressure can be reached by an advantageous vacuum desorption technique in which vapor is countercurrently withdrawn from the adsorption bed, e.g. at an initial intermediate pressure of about 15 psia and 325°F and is passed through a suitable control valve 28 associated with condenser 22 into which a controlled amount of vapor is passed for condensation by external heat exchange, as with cooling water. The water molecules in the released desorbate passing through the valve and, for example, contacting condensing tubes containing cooling water, will be caused to condense on the outer surfaces of said tubes at approximately 90°F and at a pressure of about 0.7 psia as can be determined from the vapor pressure of the desorbate. Vapor will thus be drawn through the valve by the resulting vacuum induced in condenser 22, with the pressure of said vapor in the bed being drawn down to about 1.0 psia at the end of the desorption step. By means of such a vacuum desorption technique, the pressure swing adsorption process of the invention can effectively and economically be carried out so as to achieve the desired limitation of the bed loading difference between the end of the adsorption step and the end of the desorption step. As discussed above, this

12808

limitation of the bed loading difference enables the process to be carried out with a leading heating/ adsorption front and a trailing or lagging cooling front during each adsorption step so that the heat of adsorption will be retained within the bed at a tolerable temperature level and with reasonable bed loading levels for practical commercial operations.

In an illustrative example of an ethanol drying operation carried out in accordance with the invention, a 10% ethanol-90% water by weight feed stream from a brewing vat is vaporized and passed, in the system shown in the drawing, to distillation column 3 operated at about 50 psia to produce an overhead stream approximation about 92.5% ethanol - 7.5% water by weight. The concentrated ethanol stream is passed to the two-bed pressure swing adsorption system in which one bed is on the adsorption step of the cycle while the other bed is undergoing the depressurization-desorption-repressurization portions of the overall processing sequence as cyclic adsorption operations are continuously carried out in the system.

Adsorption in each bed is carried out at about 50 psia, with the final bed pressure following the cocurrent depressurization step being about 17 psia. During the vacuum desorption step, the bed is countercurrently depressurized down to a final lower desorption pressure of about 1.0 psia. Using zeolite 3A adsorbent capable of selectively adsorbing water from the feed gas mixture, the combined product effluent from the adsorption step and from the cocurrent depressurization step will comprise about 99 weight % ethanol with the water content being reduced generally to less than about 1%.

12808

During the desorption step for each bed, the vacuum in separator 23 is conveniently maintained by removing any non-condensible gases present in the desorbate by means of small vacuum pump 24. While the condensed desorbate containing mostly water can be discarded, it will commonly contain a significant amount of ethanol such that, as continuous processing operations are continued, recycle to distillation column 3 provides a very convenient and desirable integration of the overall process of the invention. Using said zeolite 3A adsorbent such that about 90% water and 10% by weight ethanol is present in the desorbate, the desorbate stream in line 26 is conveniently passed to a heat exchanger in which the desorbate is heated for passage to column 3.

It will be appreciated that various modifications can be made in the details of the process herein described and illustrated without departing from the scope of the invention as set forth in the appended claims. Thus, as the stability of the adsorbent were to decrease in continuous long term operations, or if an adsorbent having a lower selectivity for water were employed, the recycled desorbate containing greater amounts of ethanol would desirably be passed to the portion of the distillation column wherein the ethanol-water mixture approximates the concentration of the desorbate. Similiarly, it may be economically desirable to use a lower reflux ratio in the prefractionator and then to ʰreat the resulting higher water content feed in an adsorption system having larger adsorption beds. Those skilled in the art will also appreciate that the process of the invention can be carried out using one adsorption bed, two such beds as in the illustrative example or a larger number of beds. In particular embodiments, the feed gas to the adsorption system may be passed to more than one adsorption bed at any given time in an overlapping processing sequence, and cocurrent depressurization gas can be used for pressure equalization purposes with other beds in multi-bed systems.

It should be noted that the invention should be practiced at temperatures sufficiently above the dew point of the gases being processed so as to avoid condensed capillary condensation within the pores of the adsorbent. As the concentrated ethanol-water feed mixture to the pressure swing adsorption system in the practical example given above boils at about 240°F, it is desirable to operate at a temperature suitably above this point, as at about 325°F as in the example above. The temperature rise during the exothermic adsorption reaction can be calculated as being about 110°F when a difference in loading of about 1.0% by weight is maintained. Upon desorption, the bed will be cooled back down to its original 325°F level.

While the invention has been described herein with particular emphasis on the ethanol drying application, it should be understood that various other feedstock mixtures can similarly be separated as herein disclosed and claimed. The invention is especially suited, however, for use in achieving separations of components capable of forming azeotropes or those having a relative volatility close to one. In such separations, the conventional techniques known in the art are not suitable on an overall technical and economic evaluation basis. Mixtures of water with isopropanol, sec - or tert-butanol, benzene or toluene, are illustrative of the variety of separations for which the process of the invention can be employed. In all such applications, the careful control of the difference in loading of the adsorbed component between the end of the adsorption step and the end of the desorption step will enable the heat of adsorption to be maintained and used in a manner enhancing the overall separation operation.

Thus, the careful control of the difference in loading on the bed, incorporated into the economically attractive pressure swing adsorption embodiments of the invention, enables the heat of adsorption to be retained in the bed so as to be fully available for use in completely providing the heat requirements of the cyclic process as carried out on a practical scale in commercial sized adsorption units.

Claims

1.    A cyclic adsorption process for the separation of a gas mixture containing a relatively more polar gas component comprising:

(a)    introducing the gas mixture to the feed end of a commercial size adsorption bed having a diameter of at least about one foot and containing an adsorbent capable of selectively adsorbing one such component from the other, with the non-adsorbed component being withdrawn from the product end of the bed, said introduction of the gas mixture to the bed being terminated while a relatively fast moving adsorption/heating front formed upon adsorption of the adsorbed component remains within the bed;

(b)    desorbing the adsorbed component from the bed and countercurrently discharging said desorbed component gas from the feed end of the bed, the difference in loading of the adsorbed component on the bed between the end of the adsorption step and the end of said countercurrent discharge step being maintained sufficiently low so that, during the next succeeding adsorption step (a), said adsorption/heating front will be caused to advance sufficiently ahead of a relatively slower moving, lagging cooling front that thus serves to cool only the feed end of the bed to the inlet gas mixture temperature, so that the heat of adsorption will be retained in the bed between these two fronts at a temperature tolerable to said gas mixture and to said adsorption bed; and

(c)    repeating steps (a) and (b) on a cyclic basis with additional quantities of said feed gas mixture,
whereby the retained heat of adsorption is retained in the bed for use in each succeeding desorption step, thereby enhancing the overall adsorption process and minimizing the energy requirements thereof in practical commercial operations.

2. The process of Claim 1 in which said adsorption bed is from about one to about twelve feet in diameter.

3. The process of Claim 2 in which said bed is from about two to about eight feet in diameter.

4. The process of Claim 1 in which said adsorption bed is adapted for the adsorption of the more polar component of the gas mixture.

5. The process of Claim 1 in which said desorption and countercurrent discharge of the desorbed component from the feed end of the bed are accomplished by the passing of a non-sorbable purge gas to the product end thereof at a temperature and pressure essentially the same as the temperature and pressure at the feed end of the bed, with said purge gas and said desorbed component being discharged from the feed end of the bed.

6. The process of Claim 5 in which said adsorption bed is from about one to about twelve feet in diameter.

7. The process of Claim 5 in which said adsorption bed is adapted for the adsorption of the more polar component of the gas mixture.

8. The process of Claim 5 in which said adsorption bed is adapted for the adsorption of the less polar gas component.

9. The process of Claim 5 in which the components of the gas admixture are components capable of forming an azeotrope.

10. The process of Claim 9 in which said more polar component is water and said less polar component comprises one or more organic compounds, said admixture containing at least about 2.5 weight % water.

11. The process of Claim 10 in which said organic compounds comprise primary alcohols containing from 2 to 5 carbon atoms.

12. The process of Claim 11 in which said admixture comprises water and ethanol.

13. The process of Claim 12 in which said admixture comprises from about 2.5 to about 30% by weight water, said adsorption bed being adapted for the adsorption of water.

14. The process of Claim 13 in which the difference in loading of water on the bed between the end of the adsorption step and the end of said countercurrent release and discharge step is from about 0.1 to about 3.4 lb. per 100 lb. of dry adsorbent.

15. The process of Claim 14 in which said loading difference is from about 0.5 to about 2.5 lb. of water per 100 lb. of dry adsorbent so as to moderate the exothermic temperature rise in the bed during each adsorption step (a).

16. The process of Claim 5 in which the components of the gas admixture are components having a relative volatility close to one.

17. The process of Claim 1 in which said gas mixture is introduced to the feed end of the adsorption bed at a higher adsorption pressure and in which said desorption and countercurrent discharge of the desorbed component from the feed end of the bed are accomplished by countercurrently depressurizing the bed so as to release said adsorbed component from the adsorbent and to discharge said released component from the feed end of the bed, thereby depressurizing the bed to a lower final desorption pressure, said final desorption pressure being sufficiently high as to assure achieving the desired limitation on the difference in loading of the adsorbed component on the bed.

18. The process of Claim 17 and including, prior to said countercurrent depressurization, cocurrently depressurizing the bed to an intermediate pressure so as to release void space gas comprising non-adsorbed component from the discharge end of the bed, thereby advancing said adsorption/ heating from toward but not past the discharge end of the bed.

19. The process of Claim 18 and including repressurizing the bed, with said non-adsorbed component, from the final lower desorption pressure to the higher adsorption pressure.

20. The process of Claim 19 in which said adsorption bed is from about one to about twelve feet in diameter.

21. The process of Claim 20 in which said bed is from about two to about eight feet in diameter.

22. The process of Claim 19 in which the adsorption bed is adapted for the adsorption of the less polar component of the gas mixture.

23. The process of Claim 19 in which the adsorption bed is adapted for the adsorption of the more polar component of the gas mixture.

24. The process of Claim 23 in which the components of the gas mixture are components capable of forming an azeotrope.

25. The process of Claim 24 in which said more polar component is water and said less polar component comprises one or more organic compounds, said admixture containing at least about 2.5 weight % water.

26. The process of Claim 25 in which said organic compounds comprise primary alcohols containing from 2 to 5 carbon atoms.

27. The process of Claim 26 in which said admixture comprises water and ethanol.

28. The process of Claim 27 in which said admixture comprises from about 2.5 to about 30% by weight water.

- 33 -

29. The process of Claim 28 in which said adsorption pressure is from about 35 to about 100 psia and said final lower desorption pressure is from about 0.1 to 10 psia.

30. The process of Claim 29 in which the bed is cocurrently depressurized to an intermediate pressure level of about 10 to about 50 psia.

31. The process of Claim 30 in which the feed gas mixture comprises generally about 92.5 wt. % ethanol-7.5 wt. % water and the difference in loading of water on the bed between the end of the adsorption step and the end of said countercurrent discharge step is from about 0.1 to about 3.4 lb. per 100 lb. of dry adsorbent, said adsorption bed containing essentially undiluted adsorbent having a heat capacity generally of about 0.25 BTU/lb. of adsorbent.

32. The process of Claim 31 in which said loading difference is from about 0.5 to about 2.5 lb. per 100 lb. of dry adsorbent so as to achieve reasonable loading of the bed and to ensure moderation of the exothermic temperature used in the bed during each adsorption step (a).

33. The process of Claim 23 in which the components of the gas mixture have a relative volatility close to one.

34. The process of Claim 30 in which the final lower desorption pressure in the bed is achieved by condensing the released desorbate material by external heat exchange using a cooling media in a condenser, thus inducing a vacuum pressure in the condenser equivalent to the vapor pressure of desorbate at the temperature of said cooling media.

35. The process of Claim 34 and including passing the condensed desorbate to a separator in which non-condensible gases present in the desorbate are separated from said condensed desorbate, and removing said gases from the separator by means of a vacuum pump.

36. The process of Claim 35 and including pumping the condensed desorbate from the separator to an ethanol-water distillation column capable of concentrating a dilute ethanol-water feed stream.

37. The process of Claim 36 in which the said feed stream to the distillation column contains about 10% ethanol and 90% water by weight.

38. The process of Claim 36 in which the concentrated ethanol-water stream removed from the distillation column comprises the feed gas mixture to said adsorption bed.

39. The process of Claim 38 in which the feed gas mixture comprises generally about 92.5 wt. % ethanol-7.5 wt. % water and the difference in loading of water on the bed between the end of the adsorption step and the end of said countercurrent release and discharge step is from about 0.1 to about 3.4 lb. per 100 lb. of dry adsorbent, said adsorption bed containing essentially undiluted adsorbent having a heat capacity generally of about 0.25 BTU/lb. of adsorbent -°F.

40. The process of Claim 39 in which said loading difference is from about 0.5 to about 2.5 lb. of water per 100 lb. of dry adsorbent so as to achieve reasonable loading of the bed and to ensure moderation of the exothermic temperature rise in the bed during each adsorption step (a).

41. The process of Claim 40 in which said adsorption process is about 35-50 psia, said intermediate pressure is about 10-20 psia and said final lower desorption pressure is about 1-3 psia.

0092208